(19) Europäisches Patentamt
European Patent Office
Office européen des brevets

(11) **EP 4 616 796 A1**

(12) **EUROPEAN PATENT APPLICATION**

(43) Date of publication:
**17.09.2025 Bulletin 2025/38**

(21) Application number: **25163033.1**

(22) Date of filing: **11.03.2025**

(51) International Patent Classification (IPC):
**A61B 5/024** (2006.01)     **A61B 5/145** (2006.01)
**A61B 5/00** (2006.01)

(52) Cooperative Patent Classification (CPC):
**A61B 5/02416; A61B 5/14532; A61B 5/681;
A61B 5/7203; A61B 5/7225; A61B 5/725**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **11.03.2024  US 202418601372**

(71) Applicant: **Analog Devices, Inc.
Wilmington, MA 01887 (US)**

(72) Inventor: **MCCARROLL, Benjamin John
Wilmington, 01887 (US)**

(74) Representative: **Wallin, Nicholas James
Withers & Rogers LLP
2 London Bridge
London SE1 9RA (GB)**

(54) **INTERFERENCE SUPPRESSION FILTER**

(57)     Aspects of the present disclosure include a biometric device configured to receive biometric signals from sensors placed on a subject, and attenuate at frequencies causing noise in the biometric signals. Aspects are directed to a finite impulse response (FIR) filter configured to attenuate noise frequencies caused by line frequencies and/or flicker frequencies. Aspects of the FIR filter may be configured based on an analog-to-digital (ADC) sampling rate.

*FIG. 1*

EP 4 616 796 A1

**Description**

**PRIORITY CLAIM**

The present European patent application claims priority to U.S. Non-provisional Application No. 18/601,372, entitled, "INTERFERENCE SUPPRESSION FILTER" filed March 11, 2024, which is hereby expressly incorporated by reference herein for all purposes.

**BACKGROUND**

In electrical measurements, interference, or "noise," often occurs at the line frequency or a harmonic of the line frequency. Line frequency refers to the frequency of the alternating current (AC) power that is supplied by the power grid. In North America, this frequency is typically 60Hz, while in many other parts of the world, it is 50Hz. Interference at these frequencies can distort the signal that is being measured and make it difficult to obtain accurate measurements. This is especially a problem in sensitive electronic devices, such as biometric devices used to measure small electrical signals. For example, a photoplethysmogram (PPG) device sensor may generate an analog biometric signal having a frequency ranging from 0.5 Hz to 10 Hz. In another example, a continuous glucose monitor (CGM) sensor may generate an analog biometric signal having a frequency ranging from 0.01 Hz to 1 Hz. In some examples, the biometric signals generated by such devices may be low-amp signals on the order of pico- or nano-amps. Accordingly, biometric devices may include medical devices such as PPGs, CGMs, blood oxygen sensors, electrocardiograms (ECGs), heart-rate monitors, and the like. Biometric devices may also include devices configured to measure physiological characteristics of a subject.

In the case of optical heart rate monitors and pulse oximeters, these devices work by shining light into the skin (often the finger or the wrist) of a subject and measuring the light that is reflected back. When the arteries are filled with blood, they block a small amount of light, creating a pulsing signal that can be used to measure heart rate or blood oxygen level. However, this signal is very small, often just 1% of the total signal, making it susceptible to interference from ambient light sources, including those operating at twice the line frequency (120Hz in the case of North America). This interference can cause a flickering effect in the signal, which distorts measurements. It should be noted that electro-sensitive devices are not limited to optical devices. For example, CGM and ECG devices may be susceptible to interference from ambient, artificial light sources as well as electromagnetic fields generated by nearby electrical devices. In this example, other electrical devices may cause magnetic interference and/or capacitive coupling on the subject's body which may affect electrical measurement signals of, for example, a CGM or ECG.

Analog notch filters are often used to filter out electromagnetic interference at specific frequencies. These filters "notch out" or reduce the amplitude of signals at the frequency of interest, thereby minimizing the interference. However, notch filters that operate at low frequencies, like 50Hz or 60Hz, can be large and expensive, making them unsuitable for use in small, cost-sensitive devices. Digital filters, such as digital low-pass filters, can also be used to filter out interference. These filters allow signals with a frequency lower than a certain cutoff frequency to pass through, while attenuating signals with frequencies higher than the cutoff. The sampling rate, which is the rate at which the signal is sampled or measured, must be at least twice the frequency of the signal of interest (according to the Nyquist-Shannon sampling theorem) to accurately represent the signal. However, higher sampling rates consume more power, which can be a problem in devices that run on small batteries.

Given these constraints, there exists a need for further improvements in medical and biometric device technology.

**SUMMARY**

The following presents a simplified summary of one or more aspects in order to provide a basic understanding of such aspects. This summary is not an extensive overview of all contemplated aspects and is intended to neither identify key or critical elements of all aspects nor delineate the scope of any or all aspects. Its sole purpose is to present some concepts of one or more aspects in a simplified form as a prelude to the more detailed description that is presented later.

Aspects of the disclosure are directed to a biometric device. In some examples, the biometric device may include an input port configured to receive an analog signal from a sensor, the analog signal comprising a biometric signal and an interference signal. In some examples, the biometric device may include an analog-to-digital converter (ADC) configured to convert the analog signal to a digital signal. In some examples, the biometric device may include a finite impulse response (FIR) filter characterized by at least one of a plurality of filter coefficients, the FIR filter configured to generate a filtered signal from the digital signal via attenuation of the interference signal from the digital signal, wherein the interference signal is attenuated based on a Nyquist factor and an intentional aliasing of the interference signal to at least one Nyquist frequency. In some examples, the biometric device may include an output port configured to output the filtered signal.

Aspects of the disclosure are directed to a method of signal attenuation at a biometric device. In some examples, the method includes receiving an analog signal from a sensor, the analog signal comprising a biometric signal and an interference signal. In some examples, the method includes converting the analog signal to a digital signal based on a sampling frequency. In some examples, the method includes generating a filtered signal from the digital signal via attenuation of the interference signal from the digital signal, wherein the interference signal is attenuated based on a Nyquist factor and an intentional aliasing of the interference signal to at least one Nyquist frequency. In some examples, the method includes outputting the filtered signal.

Aspects of the disclosure are directed to an apparatus for signal attenuation at a biometric device. In some examples, the apparatus includes means for receiving an analog signal from a sensor, the analog signal comprising a biometric signal and an interference signal. The means for receiving may include an input port. In some examples, the apparatus includes means for converting the analog signal to a digital signal based on a sampling frequency. The means for converting may include an analog-to-digital converter (ADC). In some examples, the apparatus includes means for generating a filtered signal from the digital signal via attenuation of the interference signal from the digital signal, wherein the interference signal is attenuated based on a Nyquist factor and an intentional aliasing of the interference signal to at least one Nyquist frequency. The means for generating may include a finite impulse response (FIR) filter. In some examples, the apparatus includes means for outputting the filtered signal. The means for outputting may include an output port.

To the accomplishment of the foregoing and related ends, the one or more aspects comprise the features hereinafter fully described and particularly pointed out in the claims. The following description and the annexed drawings set forth in detail certain illustrative features of the one or more aspects. These features are indicative, however, of but a few of the various ways in which the principles of various aspects may be employed, and this description is intended to include all such aspects and their equivalents.

**BRIEF DESCRIPTION OF THE DRAWINGS**

The disclosed aspects will hereinafter be described in conjunction with the appended drawings, provided to illustrate and not to limit the disclosed aspects, wherein like designations denote like elements, and in which:

FIG. 1 is a block diagram illustrating a schematic of an example biometric device 100 implemented as a heart rate sensor.
FIG. 2 is a compound diagram illustrating three different charts associated with the example 2-coefficient finite impulse response (FIR) filter.
FIG. 3 is a compound diagram illustrating three different charts associated with an example 3-coefficient FIR filter.
FIG. 4 is a compound diagram illustrating three different charts associated with an example 4-coefficient FIR filter.
FIG. 5 is a flow diagram illustrating an example method for attenuating noise frequencies.

**DETAILED DESCRIPTION**

The detailed description set forth below in connection with the appended drawings is intended as a description of various configurations and is not intended to represent the only configurations in which the concepts described herein may be practiced. The detailed description includes specific details for the purpose of providing a thorough understanding of various concepts. However, it will be apparent to those skilled in the art that these concepts may be practiced without these specific details. In some instances, well known structures and components are shown in block diagram form in order to avoid obscuring such concepts.

Aspects of the present disclosure are directed to a biometric device configured with a low-power, line frequency filter implemented as a finite impulse response (FIR) filter. The FIR filter may be configured to provide attenuation at 50Hz and/or 60Hz, or any other suitable line frequency. In some examples, the device may be a wearable, battery operated device. In such cases, the device may be configured as a low power device. The FIR filter, being a low-power circuit, may provide line frequency attenuation while also preserving battery power of the biometric device. Three types of FIR filters are described herein: 2-, 3-, and 4-coefficient filters. The coefficients of an FIR filter determine the filter's response to different frequencies. In some examples, the coefficients are fractions of 2. The biometric devices described herein may include cost-sensitive devices that are configured for low power consumption.

The 2-coefficient FIR filter places a null, or zero, at the Nyquist frequency. The Nyquist frequency is half the sampling rate of a discrete signal processing system. For instance, if the sampling rate is 120 samples per second (SPS), the Nyquist frequency is 60Hz. Likewise, a sampling rate of 100Hz places the null at 50Hz. This helps to eliminate noise at these frequencies. The 3-coefficient filter is a convolution of the 2-coefficient filter with itself. In this case, the frequency response of the 3-coefficient filter is the square of that of the 2-coefficient filter. This means that the 3-coefficient filter provides a more sharp and precise frequency response. The 4-coefficient filter places a null at both a half-Nyquist frequency and the Nyquist frequency. Thus, if the system samples at 4x the line frequency, a null is at both the line frequency and 2x the line frequency. This is particularly useful if artificial lights are coupling into the system and adding flicker noise. Artificial lights can cause interference at twice the line frequency in photo-sensitive sensors.

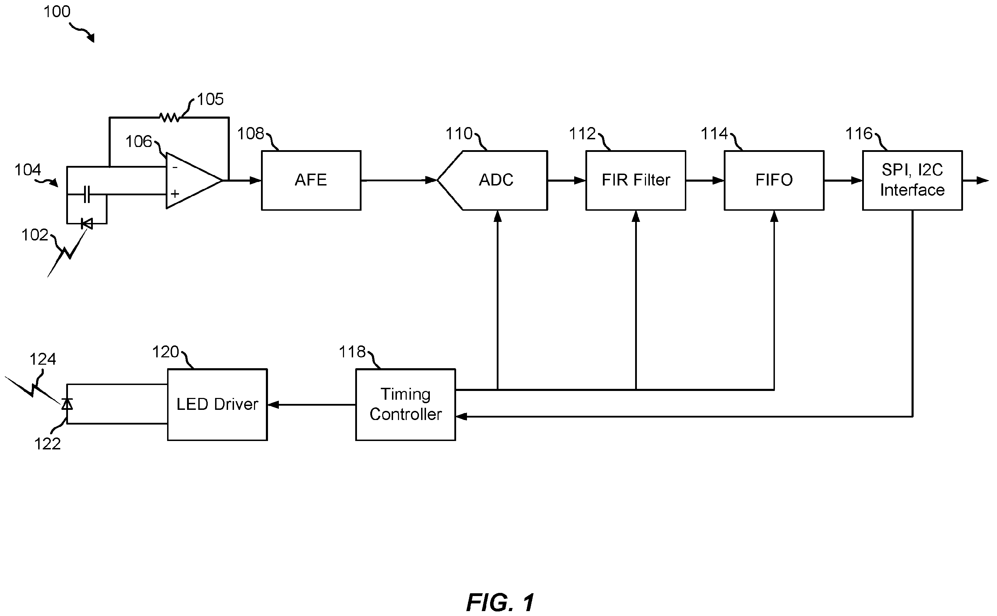

FIG. 1 is a block diagram illustrating a schematic of an example biometric device 100 implemented as a heart rate sensor (e.g., photoplethysmogram (PPG)) with an FIR filter configured to reduce or eliminate noise generated from nearby electrical devices (e.g., artificial light sources, power lines, other electronics on or in the vicinity of a subject, etc.). It should be noted that while this example is directed to a photosensitive biometric device, this example is not limiting. In other words, the FIR filter illustrated and described herein may be implemented in other biometric devices in a similar manner.

As illustrated the biometric device 100 includes a light source 122 (e.g., a light emitting diode (LED)) and an LED driver 120 configured to control and power the light source 122. In the example of a heart rate sensor, the light source 122 may be disposed over a surface of a subject's finger. Radiated light 124 from the light source 122 may pass through the subject finger. A photodiode circuit 104 may be disposed over an opposite surface of the subject's finger and configured to receive and measure an intensity of incident light. When the photodiode circuit 104 detects the incident light 102, it may generate an electrical signal having a current proportional to the intensity of the incident light. Changes in this signal may correspond to the changes in blood volume in the tissue.

The electrical signal may be passed to a transimpedance amplifier (TIA) 106 configured to convert the current from the photodiode circuit 104 (the input to the TIA 106) into a continuous voltage signal that is output from the TIA 106. The TIA 106 may include a negative feedback resistor 105 configured to provide a path for feedback from the output of the op amp back to its inverting input (-). Such a configuration may improve stabilization the gain of the op amp and prevent the op amp from going into saturation. The biometric device 100 may also include an analog front end (AFE) 108 coupled to an analog-to-digital converter (ADC) 110. The AFE 108 may receive, as input, the voltage signal from the TIA 106 and perform one or more signal conditioning techniques on the voltage signal. Typically, an AFE 108 may include analogue amplifiers, operational amplifiers, filters, sample and hold circuits, etc. In some examples, the AFE 108 may include the ADC 110 as part of its own circuitry.

The ADC 110 may be configured to receive, as an input, the continuous voltage signal (e.g., an analog signal) from the AFE 108 and output a digital signal representative of the voltage signal. Generally, the signaling generated by the photodiode circuit 104 is analog in nature, while the various integrated circuits (ICs) that form other aspects of a heart rate monitor are configured to interpret binary data, or digital signals. Hence, the ADC 110 may be configured to sample the continuous voltage signal output from the AFE 108 at a particular sampling rate based on a frequency of the interference or a line frequency that the FIR filter 112 is configured to null. As used herein, a "sampling rate" relates to a number of samples of the continuous voltage signal taken by the ADC 110 per second.

The digital signal generated by the ADC 110 may be received as an input to an FIR filter 112. The FIR filter 112 may receive the digital signal (representative of the sampled analog voltage signal form the AFE 108) and multiply each received sample by a certain weight, or coefficient before summing them to produce an output signal. The FIR filter 112 may provide a filtered signal as input to a first-in first-out (FIFO) 114 buffer, which then may output the filtered signal via an output port 116. The output port 116 may be implemented as a serial peripheral interface (SPI), an inter-integrated circuit (I2C) or any other suitable interface. For example, the output port 116 may interface with a processor or controller (e.g., microcontroller unit (MCU), application processor, etc.) of a separate device. A timing controller 118 may control a clock signal provided to various components of the biometric device 100 to ensure synchronous interoperation.

It should be noted that in some examples, the sensor (e.g., illustrated as a heart rate sensor and implemented as one or more of the photodiode circuit 104, the LED driver, and/or the light source 122) may be an external device separate from the biometric device 100. In such an example, the biometric device 100 may be configured to couple to the sensor via an input port through which the biometric device 100 is configured to receive signal(s) generated by the sensor.

*Examples of a Finite Impulse Response (FIR) Filter Implemented in a Biometric Device*

In certain aspects, an FIR filter (e.g., FIR filter 112 of FIG. 1) may be configured to attenuate a line frequency or a harmonic thereof. In the example of a biometric device (e.g., biometric device 100 of FIG. 1), the line frequency may be generated by an artificial ambient light, which is a primary source of interference for optical devices. For other devices (e.g., non-optical biometric devices), a primary source of interference may come from other, nearby electrical devices. In either example, the line frequency may be approximately 60Hz in North America, and 50Hz in other regions (e.g., Europe). Thus, the FIR filter may be configured to attenuate interfering line frequencies at one or both of 60Hz or 50Hz.

In certain aspects, an FIR filter may be configured as a 2-, 3-, or 4-coefficient filter. An example 2-coefficient filter characterized by Equation 1 below. Note that in Equation 1, the 0.5 value may be changed to any suitable value. For example, 0.5 may be changed to 1 in order for DC gain to be unity.

$$y[n] = 0.5(x[n] + x[n - 1]) \qquad \qquad \text{Equation 1}$$

Where:

y[n] is a current output of the FIR filter,
x[n] is a current input (e.g., digital sample from an ADC) to the filter, and
x[n-1] is a previous input to the filter.

Note that because the DC gain must be one, the sum of all the coefficients may be one. As described in Equation 1, the FIR filter adds the current input (x[n]) and the previous input (x[n-1]), then multiplies the result by 0.5. In other words, the FIR filter averages the current and previous inputs. Because the filter operates on two consecutive samples, it may be effective at attenuating frequencies at or above a Nyquist frequency. This is because signals at the Nyquist frequency alternate their polarity every sample, and signals above the Nyquist frequency alternate their polarity more than once per sample. When these signals are averaged, they tend towards zero, effectively reducing their contribution to the output signal. In effect, the FIR filter may reduce high-frequency components (e.g., 50/60Hz line frequencies) of a signal output from an ADC so that noise distortion of low frequency components is reduced or eliminated. It should be noted that the Nyquist frequency is defined as half of a sampling rate. So, if the sampling rate of the ADC is 120Hz, then the Nyquist frequency would be 60Hz. Thus, in the example of a biometric device used in North America, the ADC sampling rate may be set to 120Hz (e.g., 120 samples per second) so that the FIR filter may cause a null at the 60Hz Nyquist frequency. In other regions, the ADC sampling rate may be set to 100Hz (e.g., 100 samples per second) so that the FIR filter may cause a null at the 50Hz Nyquist frequency.

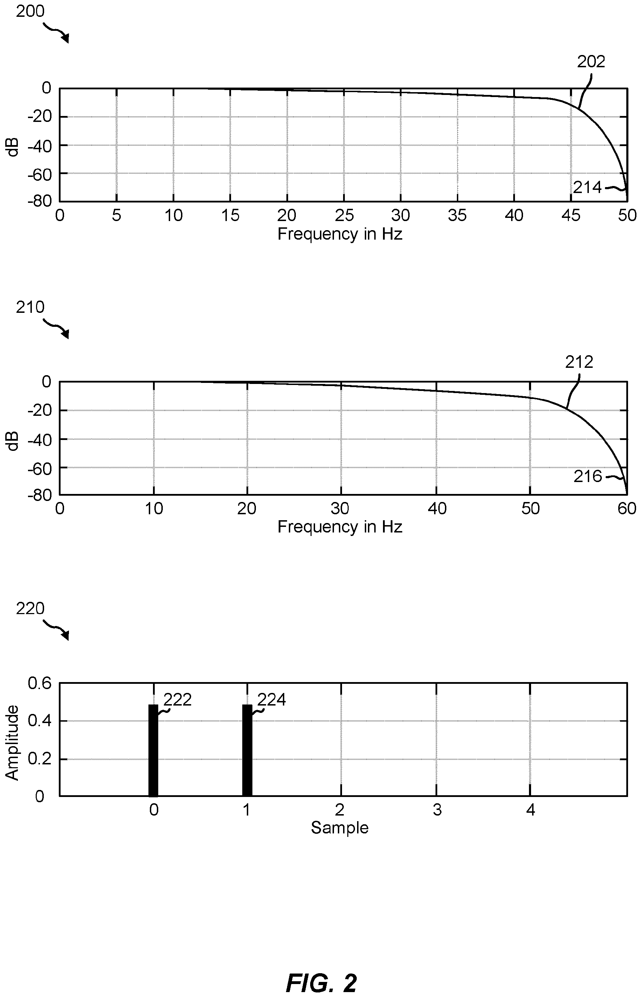

FIG. 2 is a compound diagram illustrating three different charts associated with the example 2-coefficient filter characterized by Equation 1. A first chart 200 and a second chart 210 illustrate an advancing decline in decibels (dBs) as a frequency increases to line frequencies 50Hz and 60Hz, respectively. In other words, the first chart 200 and the second chart 210 show frequency responses of a 2-ceofficient FIR filter applied to a 50Hz line frequency and applied to a 60 Hz line frequency. The signals illustrated in these charts are signals output by an example 2-coefficient FIR filter for which the input signal is generated by an ADC (e.g., ADC 110 of FIG. 1).

With regard to the first chart 200, a first signal 202 output by an associated FIR filter has a null 214 at a Nyquist frequency of 50Hz in order to filter out interference caused by artificial light and/or nearby electronics. In this example, the sampling rate of the ADC may be 100Hz, or 100 samples per second. With regard to the second chart 210, a second signal 212 output by an associated FIR filter has a null 216 at a Nyquist frequency of 60Hz in order to filter out interference caused by artificial light and/or nearby electronics. In this example, the sampling rate of the ADC may be 120Hz, or 120 samples per second.

As noted above, biometric devices (e.g., biometric device 100 of FIG. 1) may be used to measure signals that are very small and have relatively low frequencies. Thus, although the first chart 200 and the second chart 210 show a dB roll-off starting around the 15Hz level and higher, signals measured by the biometric devices often have a frequency lower than 15Hz. Accordingly, the FIR filters may have little to no effect on the measured signal.

A third chart 220 illustrates an impulse response of the example 2-coefficient FIR filter for given discrete-time signals or impulse signals (e.g., x[n]) output by the ADC. As used herein, an impulse response is the output (e.g., y[n]) of the FIR filter based on the impulse signals that are input to the FIR filter. The y-axis relates to signal amplitude, while the x-axis refers to a time index or a sequence of impulse response signals (e.g., y[n]) output from the FIR filter. Here, it should be noted that the FIR filter operates on discrete-time signals output by the ADC. Because the ADC operates by sampling a continuous-time signal at a certain rate, the discrete-time signals generated by the ADC are a sequence of quantities where each quantity is defined by a specific moment in time. Thus, using the example FIR filter defined by Equation 1 above, a first impulse response 222 may be produced by summing a first impulse signal of time n-3 and a second impulse signal of time n-2 (e.g., contiguous impulse signals that occur prior to a current time), then multiplying the sum by 0.5. A second impulse response 224 may be produced by summing a third impulse signal of time n-1 and a fourth impulse signal of time n (where n is the current time).

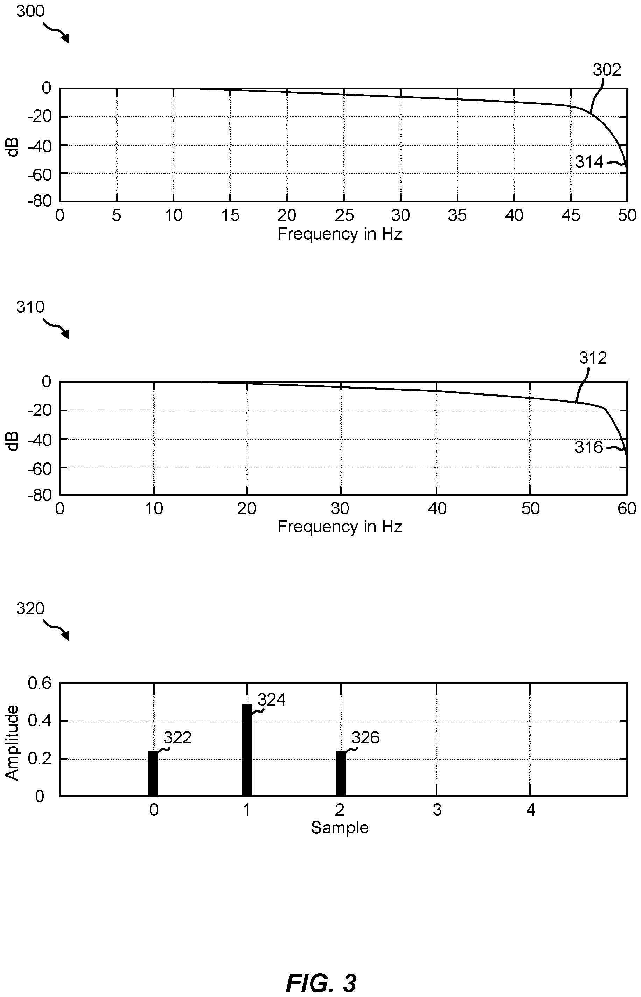

FIG. 3 is a compound diagram illustrating three different charts associated with an example 3-coefficient filter characterized by Equation 2 below. Note that in Equation 2, one or more of the 0.5 value and 0.25 values may be changed to any suitable value(s).

$$y[n] = 0.25(x[n]) + 0.5(x[n-1]) + 0.25(x[n-2]) \qquad \text{Equation 2}$$

A first chart 300 and a second chart 310 show frequency responses of a 3-coefficient FIR filter applied to a 50Hz line frequency and applied to a 60 Hz line frequency, respectively. The signals illustrated in these charts are signals output by an example 3-coefficient FIR filter for which the input signal is generated by an ADC.

With regard to the first chart 300, a first signal 302 output by an associated FIR filter has a null 314 at a Nyquist frequency of 50Hz in order to filter out interference caused by artificial light and/or nearby electronics. In this example, the sampling rate of the ADC may be 100Hz, or 100 samples per second. With regard to the second chart 310, a second signal 312 output by an associated FIR filter has a null 316 at a Nyquist frequency of 60Hz in order to filter out interference caused by artificial light and/or nearby electronics. In this example, the sampling rate of the ADC may be 120Hz, or 120 samples per second.

As noted above, biometric devices (e.g., biometric device 100 of FIG. 1) may be used to measure signals that are very small and have relatively low frequencies. Thus, although the first chart 300 and the second chart 310 show a dB roll-off starting around the 15Hz level and higher, signals measured by the biometric devices often have a frequency lower than 15Hz. Accordingly, the FIR filters may have little to no effect on the measured signal.

A third chart 320 illustrates an impulse response of the example 3-coefficient FIR filter for given discrete-time signals or impulse signals output by the ADC. The y-axis relates to signal amplitude, while the x-axis refers to a time index or a sequence of impulse response signals (e.g., y[n]) output from the FIR filter. Using the example FIR filter defined by Equation 2 above, a first impulse response 322 may be produced by summing a first impulse signal of time n-1 and a second impulse signal of time n, then multiplying the sum by 0.5. Here, the first impulse signal and the second impulse signal are contiguous impulse signals that occur prior to a current time (n-1) and a current time (n). A second impulse response 324 and a third impulse response 326 may be generated by the FIR filter based on subsequent impulse signals input to the FIR filter by the ADC and using the operations described in relation to the first impulse response 322.

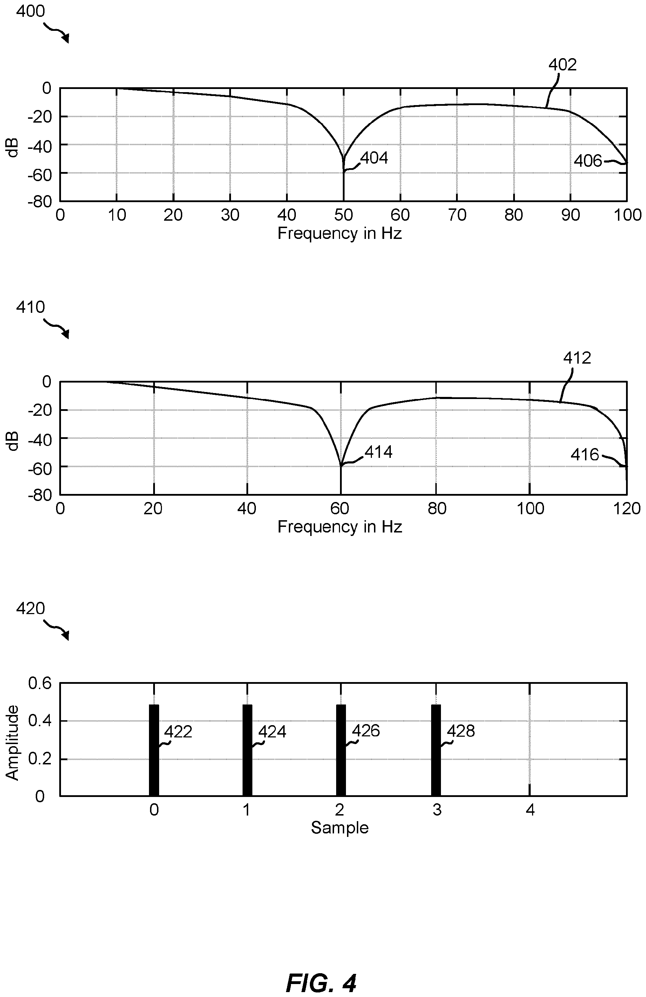

FIG. 4 is a compound diagram illustrating three different charts associated with an example 4-coefficient filter characterized by Equation 3 below. Note that in Equation 3, the 0.25 value may be changed to any suitable value.

$$y[n] = 0.25(x[n] + x[n-1] + x[n-2] + x[n-3]) \qquad \text{Equation 3}$$

A first chart 400 and a second chart 410 show frequency responses of a 4-ceofficient FIR filter applied to a 50Hz line frequency and applied to a 60 Hz line frequency, respectively. Here, for example, a 4-coefficient FIR filter may find applicability if light flicker associated with an artificial light couples with the biometric device. In this case, the line frequency associated with the light may be 50Hz or 60Hz, but the flicker component may be twice the line frequency (e.g., 100Hz or 120Hz). Accordingly, the flicker component may be set as the Nyquist frequency. The Nyquist frequency is half the sampling rate associated with the signal input into the FIR filter. Thus, the Nyquist frequency for the flicker component, which is twice the line frequency, may be defined as follows:

$$f_S = 4(f_L) \qquad \text{Equation 4}$$

Where:

$f_s$ is the sampling rate associated with the ADC signal input to the FIR filter, and
$f_L$ is the line frequency.

Accordingly, if the sampling rate is four times the line frequency, the 4-coefficient FIR filter may place a null at the Nyquist frequency (associated with the flicker component) and at half the Nyquist frequency (associated with the line frequency). Thus, for a line frequency of 60Hz, the sampling rate should be 240Hz.

With regard to the first chart 400, a first signal 402 output by the example 4-coefficient FIR filter has a first null 404 at half the Nyquist frequency in order to filter out a 50Hz line frequency generated by artificial light and/or nearby electronics. The first signal 402 also places a second null 406 at the Nyquist frequency in order to filter out a 100Hz flicker component associated with the light.

With regard to the second chart 410, a first signal 412 output by the example 4-coefficient FIR filter has a first null 414 at half the Nyquist frequency in order to filter out a 60Hz line frequency and a second null 416 at the Nyquist frequency in order to filter out a 120Hz flicker component.

A third chart 420 illustrates an impulse response of the example 4-coefficient FIR filter for given discrete-time signals output by the ADC. The y-axis relates to signal amplitude, while the x-axis refers to a time index or a sequence of impulse response signals (e.g., y[n]) output from the FIR filter. Using the example FIR filter defined by Equation 3 above, a first impulse response 422 may be produced by summing a first impulse signal of time n, a second impulse signal of time n-1, a third impulse signal of time n-2, and a fourth impulse signal of time n-3, then multiplying the sum by 0.25. A second impulse response 424, a third second impulse response 426, and a fourth impulse response 428 may be produced by summing subsequent impulse signals and averaging the sum as described for the first impulse response 422.

In certain aspects, a FIR filter may be configured to use aliasing by setting the Nyquist frequency to the line frequency divided by an odd integer. Thus, building on Equation 4 above, a sampling rate may be determined as follows:

$$\{f_S\} = \frac{4(f_L)}{\{1, 3, 5, 7, 9, \ldots\}} \qquad \text{Equation 5}$$

By taking advantage of aliasing, an FIR filter may still effectively filter one or more of a line frequency and/or a flicker component despite using a lower sampling rate relative to the examples above. It should also be noted that the aliasing does not affect the biometric signal because the frequency or bandwidth of the biometric signal is low enough to be unaffected by the aliasing. Moreover, the lower sampling rate may reduce power consumption of a biometric device configured with an ADC and FIR filter. For example, in a scenario where the line frequency is 60Hz and the flicker component is 120Hz, the sampling rate should be 240Hz if the flicker component is set at the Nyquist frequency. However, in an example case where the 240Hz sampling rate is divided by odd integer 3, the new sampling rate is 80Hz. Thus, in this example, because the flicker component is at 120Hz and the sampling rate is at 80Hz, an alias tone may be generated at 40Hz (e.g., the difference between the flicker component frequency and the sampling rate) which is the Nyquist frequency (half of 80Hz).

It should be noted that in some examples, the biometric device may be configured as an electromagnetic device (e.g., ECG, CGM, and the like) as opposed to an optical device (e.g., PPG device and the like). As such, the electromagnetic biometric device may not be as affected by the flicker component as an optical biometric device. Thus, without the flicker component at twice the line frequency, the sampling rate may be defined as follows:

$$f_S = 2(f_L) \qquad \text{Equation 6}$$

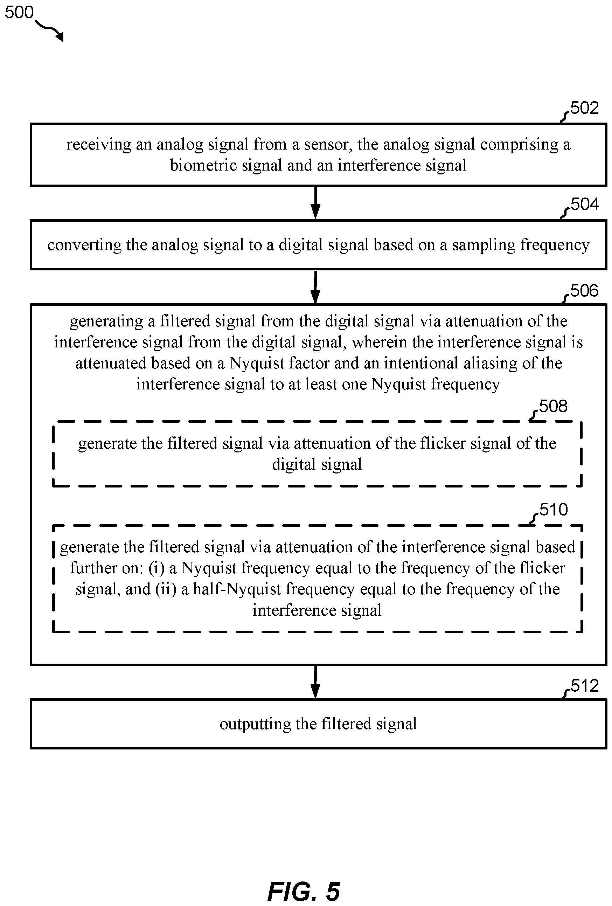

Turning to FIG. 5, a method 500 for signal attenuation may be implemented by the biometric device 100 of FIG. 1 and one or more components of the biometric device 100.

At 502, the method 500 may include receiving an analog signal from a sensor, the analog signal comprising a biometric signal and an interference signal. For example, the sensor may include the photodiode circuit 104 of FIG. 1 or non-photo-sensitive sensors such as electrodes for measuring glucose (e.g., as part of a continuous glucose monitor (CGM)) or measuring heart rate/rhythm (e.g., as part of an electrocardiogram (ECG or EKG)). It should be noted that these examples are not limiting, and the method 500 for signal attenuation may be implemented on any suitable device. The interference signal may be a noise signal that is included in the analog signal obtained from the sensor. For example, the interference signal may include noise at a line frequency.

In some examples, the analog signal and the biometric and interference aspects of that signal form continuous, analog signal. In the applications described above, the biometric signal may be implemented as a very small current (e.g., on the order of micro-amps). The interference signal may be caused by one or more of a line frequency or a flicker signal associated with a line frequency. In some examples, the line frequency may include a 50Hz or 60Hz signal generated by an electrical device separate from the biometric device. This 50Hz or 60Hz signal may introduce noise to the biometric signal. Moreover, photosensitive sensors may be susceptible to signal noise due to a stroboscopic effect of an artificial light source. For example, incandescent and LED lights typically flicker at a high frequency (e.g., 100Hz/120Hz) due to their power source being AC or pulsed DC.

At 504, the method 500 may include converting the analog signal to a digital signal based on a sampling frequency. For example, the biometric device 100 may include an ADC (e.g., ADC 110 of FIG. 1) configured to convert the analog signal using a particular sampling rate. It should be noted that the sampling rate of the ADC 110 may be a function of one or more of the line frequency, a frequency associated with the flicker signal, a Nyquist factor, and/or whether the FIR filter (e.g., FIR filter 112 of FIG. 1) is configured as a 2-coefficient filter, a 3-coefficient filter, or a 4-coefficient filter.

At 506, the method 500 may include generating a filtered signal from the digital signal via attenuation of the interference signal from the digital signal, wherein the interference signal is attenuated based on a Nyquist factor and an intentional aliasing of the interference signal to at least one Nyquist frequency. For example, the FIR filter 112 may be used to attenuate the line frequency aspect of the digital signal so that the biometric signal is clearer. The Nyquist factor may include an odd integer such as any of 1, 3, 5, 7, 9, and on, as shown in Equation 5. By dividing a noise frequency (e.g., flicker frequency or line frequency) by the Nyquist factor, a sampling rate for the ADC 110 may be determined.

At 508, the method 500 may include generating the filtered signal via attenuation of the flicker signal of the digital signal. For example, the FIR filter 112 may generate the filtered signal by attenuating both the flicker frequency and the line frequency.

At 510, the method 500 may include generating the filtered signal via attenuation of the interference signal based further on: (i) a Nyquist frequency equal to the frequency of the flicker signal, and (ii) a half-Nyquist frequency equal to the frequency of the interference signal. Here, the sampling rate of the ADC 110 may be set so that the Nyquist frequency of the FIR filter 112 is equal to the flicker frequency, and the Nyquist frequency divided by two is equal to the line frequency.

At 512, the method 500 may include outputting the filtered signal. For example, the filtered signal may be output (e.g., via the output port 116 of FIG. 1) to a monitor or a processing system for further computations.

In certain aspects, the interference signal is associated with an ambient line frequency of one or more electronic devices separate from the biometric device 100.

In certain aspects, the Nyquist factor is an integer divisor of the ambient line frequency, wherein the intentional aliasing is a result of dividing the ambient line frequency by the integer divisor, and wherein a sampling frequency of the ADC is a function of the result of dividing the ambient line frequency.

In certain aspects, the analog signal further comprises a flicker signal, wherein a frequency of the flicker signal is twice a frequency of the ambient line frequency, and wherein the method further comprises generating the filtered signal via attenuation of the flicker signal of the digital signal.

In certain aspects, a sampling frequency of the ADC is set to two-times or four-times the frequency of the interference signal.

In certain aspects, the frequency of the flicker signal is twice the frequency of the ambient line frequency.

In certain aspects, the sensor is an optical sensor (e.g., photodiode circuit 104 of FIG. 1), wherein the flicker signal is associated with a stroboscopic effect of an artificial light source, and wherein the interference signal is associated with a line frequency of at least one of the artificial light source or one or more electronic devices separate from the biometric device

In certain aspects, the frequency of the flicker signal is twice the frequency of the ambient line frequency.

In certain aspects, a sampling rate of the ADC is a function of a frequency of the interference signal.

In certain aspects, the biometric signal is a photoplethysmography (PPG) signal or a glucose measuring signal, and wherein the biometric signal has a smaller bandwidth relative to a frequency of the interference signal.

In certain aspects, the Nyquist factor is an odd integer.

In certain aspects, a frequency of the interference signal is 50Hz or 60 Hz.

In certain aspects, the FIR filter 112 is characterized by three filter coefficients based on a convolution of another FIR filter characterized by two filter coefficients.

*Additional Considerations*

The above detailed description set forth above in connection with the appended drawings describes examples and does not represent the only examples that may be implemented or that are within the scope of the claims. The term "example," when used in this description, means "serving as an example, instance, or illustration," and not "preferred" or "advantageous over other examples." The detailed description includes specific details for the purpose of providing an understanding of the described techniques. These techniques, however, may be practiced without these specific details. For example, changes may be made in the function and arrangement of elements discussed without departing from the scope of the disclosure. Also, various examples may omit, substitute, or add various procedures or components as appropriate. For instance, the methods described may be performed in an order different from that described, and various steps may be added, omitted, or combined. Also, features described with respect to some examples may be combined in other examples. In some instances, well-known structures and apparatuses are shown in block diagram form in order to avoid obscuring the concepts of the described examples.

Information and signals may be represented using any of a variety of different technologies and techniques. For example, data, instructions, commands, information, signals, bits, symbols, and chips that may be referenced throughout the above description may be represented by voltages, currents, electromagnetic waves, magnetic fields or particles, optical fields or particles, computer-executable code or instructions stored on a computer-readable medium, or any combination thereof.

Several aspects of biometric devices are presented herein, with reference to various apparatus and methods. These apparatus and methods are described in the detailed description and illustrated in the accompanying drawings by various blocks, components, circuits, processes, algorithms, etc. (collectively referred to as "elements"). These elements may be implemented using electronic hardware, computer software, or any combination thereof. Whether such elements are implemented as hardware or software depends upon the particular application and design constraints imposed on the overall system.

Accordingly, in one or more example embodiments, the functions described may be implemented in hardware, software, or any combination thereof. If implemented in software, the functions may be stored on or encoded as one or more instructions or code on a computer-readable medium. Computer-readable media includes computer storage media. Storage media may be any available media that may be accessed by a computer. By way of example, and not limitation, such computer-readable media may comprise a random-access memory (RAM), a read-only memory (ROM), an electrically erasable programmable ROM (EEPROM), optical disk storage, magnetic disk storage, other magnetic storage devices, combinations of the aforementioned types of computer-readable media, or any other medium that may be used to store computer executable code in the form of instructions or data structures that may be accessed by a computer.

The various illustrative blocks and components described in connection with the disclosure herein may be implemented or performed with a specially-programmed device, such as but not limited to a processor, a digital signal processor (DSP), an ASIC, a FPGA or other programmable logic device, a discrete gate or transistor logic, a discrete hardware component, or any combination thereof designed to perform the functions described herein. A specially-programmed processor may be a microprocessor, but in the alternative, the processor may be any conventional processor, controller, microcontroller, or state machine. A specially-programmed processor may also be implemented as a combination of computing devices, e.g., a combination of a DSP and a microprocessor, multiple microprocessors, one or more microprocessors in conjunction with a DSP core, or any other such configuration.

The functions described herein may be implemented in hardware, software executed by a processor, firmware, or any combination thereof. If implemented in software executed by a processor, the functions may be stored on or transmitted over as one or more instructions or code on a non-transitory computer-readable medium. Other examples and implementations are within the scope and spirit of the disclosure and appended claims. For example, due to the nature of software, functions described above may be implemented using software executed by a specially programmed processor, hardware, firmware, hardwiring, or combinations of any of these. Features implementing functions may also be physically located at various positions, including being distributed such that portions of functions are implemented at different physical locations. Also, as used herein, including in the claims, "or" as used in a list of items prefaced by "at least one of" indicates a disjunctive list such that, for example, a list of "at least one of A, B, or C" means A or B or C or AB or AC or BC or ABC (i.e., A and B and C).

The previous description of the disclosure is provided to enable a person skilled in the art to make or use the disclosure. Various modifications to the disclosure will be readily apparent to those skilled in the art, and the common principles defined herein may be applied to other variations without departing from the spirit or scope of the disclosure. Furthermore, although elements of the described aspects may be described or claimed in the singular, the plural is contemplated unless limitation to the singular is explicitly stated. Additionally, all or a portion of any aspect may be utilized with all or a portion of any other aspect, unless stated otherwise. Thus, the disclosure is not to be limited to the examples and designs described herein but is to be accorded the widest scope consistent with the principles and novel features disclosed herein.

*Example Aspects*

Aspect 1 is a method of signal attenuation at a biometric device, comprising: receiving an analog signal from a sensor, the analog signal comprising a biometric signal and an interference signal; converting the analog signal to a digital signal based on a sampling frequency; generate a filtered signal from the digital signal via attenuation of the interference signal from the digital signal, wherein the interference signal is attenuated based on a Nyquist factor and an intentional aliasing of the interference signal to at least one Nyquist frequency.

Aspect 2 is the method of aspect 1, wherein the interference signal is associated with an ambient line frequency of one or more electronic devices separate from the biometric device.

Aspect 3 is the method of any of aspects 1 and 2, wherein the Nyquist factor is an integer divisor of the ambient line frequency, wherein the intentional aliasing is a result of dividing the ambient line frequency by the integer divisor, and wherein the sampling frequency is a function of the result of dividing the ambient line frequency.

Aspect 4 is the method of any of aspects 1-3, wherein the analog signal further comprises a flicker signal, wherein a frequency of the flicker signal is twice a frequency of the ambient line frequency, and wherein the method further comprises generating the filtered signal via attenuation of the flicker signal of the digital signal.

Aspect 5 is the method of aspect 4, further comprising generating the filtered signal via attenuation of the interference signal based further on: (i) a Nyquist frequency equal to the frequency of the flicker signal, and (ii) a half-Nyquist frequency equal to the frequency of the interference signal.

Aspect 6 is the method of aspect 4, wherein the sampling frequency is set to two-times or four-times the frequency of the interference signal.

Aspect 7 is the method of aspect 4, wherein the biometric signal is a photoplethysmography (PPG) signal, wherein the flicker signal is associated with a stroboscopic effect of an artificial light source, and wherein the interference signal is associated with a line frequency of at least one of the artificial light source or one or more electronic devices separate from the biometric device.

Aspect 8 is the method of claim 4, wherein the frequency of the flicker signal is twice the frequency of the ambient line frequency.

Aspect 9 is the method of any of aspects 1-8, wherein a sampling rate of the ADC is a function of a frequency of the interference signal.

Aspect 10 is the method of any of aspects 1-9, wherein the biometric signal is a photoplethysmography (PPG) signal or a glucose measuring signal, and wherein the biometric signal has a smaller bandwidth relative to a frequency of the interference signal.

Aspect 11 is the method of any of aspects 1-10, wherein the Nyquist factor is an odd integer.

Aspect 12 is the method of any of aspects 1-11, wherein a frequency of the interference signal is 50Hz or 60 Hz.

Aspect 13 is the method of any of aspects 1-12, wherein the FIR filter is characterized by three filter coefficients based on a convolution of another FIR filter characterized by two filter coefficients.

Aspect 14 is an apparatus for frequency attenuation comprising an ADC and an FIR filter configured to perform a method in accordance with any one of aspects 1-13.

Aspect 15 is an apparatus for frequency attenuation comprising means for performing a method in accordance with any one of aspects 1-13.

There follows a list of numbered features defining particular embodiments of the present disclosure. Where a numbered feature refers to one or more earlier numbered features then those features should be considered in combination.

1. A biometric device, comprising:

   an input port configured to receive an analog signal from a sensor, the analog signal comprising a biometric signal and an interference signal;
   an analog-to-digital converter (ADC) configured to convert the analog signal to a digital signal;
   a finite impulse response (FIR) filter characterized by at least one of a plurality of filter coefficients, the FIR filter configured to generate a filtered signal from the digital signal via attenuation of the interference signal from the digital signal, wherein the interference signal is attenuated based on a Nyquist factor and an intentional aliasing of the interference signal to at least one Nyquist frequency; and
   an output port configured to output the filtered signal.

2. The biometric device of feature 1, wherein the interference signal is associated with an ambient line frequency of one or more electronic devices separate from the biometric device.

3. The biometric device of feature 2, wherein the Nyquist factor is an integer divisor of the ambient line frequency, wherein the intentional aliasing is a result of dividing the ambient line frequency by the integer divisor, and wherein a sampling frequency of the ADC is a function of the result of dividing the ambient line frequency.

4. The biometric device of features 2 or 3, wherein the analog signal further comprises a flicker signal, wherein a frequency of the flicker signal is twice a frequency of the ambient line frequency, and wherein the FIR filter is further configured to generate the filtered signal via attenuation of the flicker signal of the digital signal.

5. The biometric device of feature 4, wherein the FIR filter is characterized by a 4-coefficient filter of the plurality of filter coefficients, and wherein the FIR filter is further configured to generate the filtered signal via attenuation of the interference signal based further on: (i) a Nyquist frequency equal to the frequency of the flicker signal, and (ii) a half-Nyquist frequency equal to the frequency of the interference signal.

6. The biometric device of features 4 or 5, wherein a sampling frequency of the ADC is set to two-times or four-times the frequency of the interference signal.

7. The biometric device of any of features 4 to 6, wherein the sensor is an optical sensor, wherein the flicker signal is associated with a stroboscopic effect of an artificial light source, and wherein the interference signal is associated with a line frequency of at least one of the artificial light source or one or more electronic devices separate from the biometric device.

8. The biometric device of any of features 4 to 7, wherein the frequency of the flicker signal is twice the frequency of the ambient line frequency.

9. The biometric device of any of features 1 to 8, wherein a sampling rate of the ADC is a function of a frequency of the interference signal.

10. The biometric device of any of features 1 to 9, wherein the biometric signal is a photoplethysmography (PPG) signal or a glucose measuring signal, and wherein the biometric signal has a smaller bandwidth relative to a frequency of the interference signal.

11. The biometric device of any of features 1 to 10, wherein the Nyquist factor is an odd integer.

12. The biometric device of any of features 1 to 11, wherein a frequency of the interference signal is 50Hz or 60 Hz.

13. The biometric device of any of features 1 to 12, wherein the FIR filter is characterized by three filter coefficients based on a convolution of another FIR filter characterized by two filter coefficients.

14. A method of signal attenuation at a biometric device, comprising:

receiving an analog signal from a sensor, the analog signal comprising a biometric signal and an interference signal;
converting the analog signal to a digital signal based on a sampling frequency;
generating a filtered signal from the digital signal via attenuation of the interference signal from the digital signal, wherein the interference signal is attenuated based on a Nyquist factor and an intentional aliasing of the interference signal to at least one Nyquist frequency; and
outputting the filtered signal.

15. The method of feature 14, wherein the interference signal is associated with an ambient line frequency of one or more electronic devices separate from the biometric device.

16. The method of feature 15, wherein the Nyquist factor is an integer divisor of the ambient line frequency, wherein the intentional aliasing is a result of dividing the ambient line frequency by the integer divisor, and wherein the sampling frequency is a function of the result of dividing the ambient line frequency.

17. The method of features 15 or 16, wherein the analog signal further comprises a flicker signal, wherein a frequency of the flicker signal is twice a frequency of the ambient line frequency, and wherein the method further comprises generating the filtered signal via attenuation of the flicker signal of the digital signal.

18. The method of feature 17, wherein the flicker signal is associated with a stroboscopic effect of an artificial light source.

19. The method of features 17 or 18, wherein the sampling frequency is set to two-times or four-times the frequency of the interference signal.

20. The method of any of features 14 to 19, wherein the sampling frequency is a function of a frequency of the interference signal.

## Claims

1. A biometric device, comprising:

an input port configured to receive an analog signal from a sensor, the analog signal comprising a biometric signal and an interference signal;
an analog-to-digital converter (ADC) configured to convert the analog signal to a digital signal;
a finite impulse response (FIR) filter **characterized by** at least one of a plurality of filter coefficients, the FIR filter configured to generate a filtered signal from the digital signal via attenuation of the interference signal from the digital signal, wherein the interference signal is attenuated based on a Nyquist factor and an intentional aliasing of the interference signal to at least one Nyquist frequency; and
an output port configured to output the filtered signal.

2. The biometric device of claim 1, wherein the interference signal is associated with an ambient line frequency of one or more electronic devices separate from the biometric device.

3. The biometric device of claim 2, wherein the Nyquist factor is an integer divisor of the ambient line frequency, wherein the intentional aliasing is a result of dividing the ambient line frequency by the integer divisor, and wherein a sampling frequency of the ADC is a function of the result of dividing the ambient line frequency.

4. The biometric device of claims 2 or 3, wherein the analog signal further comprises a flicker signal, wherein a frequency of the flicker signal is twice a frequency of the ambient line frequency, and wherein the FIR filter is further configured to generate the filtered signal via attenuation of the flicker signal of the digital signal.

**5.** The biometric device of claim 4, wherein the FIR filter is **characterized by** a 4-coefficient filter of the plurality of filter coefficients, and wherein the FIR filter is further configured to generate the filtered signal via attenuation of the interference signal based further on: (i) a Nyquist frequency equal to the frequency of the flicker signal, and (ii) a half-Nyquist frequency equal to the frequency of the interference signal.

**6.** The biometric device of claims 4 or 5, wherein:

a) a sampling frequency of the ADC is set to two-times or four-times the frequency of the interference signal; and/or
b) the frequency of the flicker signal is twice the frequency of the ambient line frequency

**7.** The biometric device of any of claims 4 to 6, wherein the sensor is an optical sensor, wherein the flicker signal is associated with a stroboscopic effect of an artificial light source, and wherein the interference signal is associated with a line frequency of at least one of the artificial light source or one or more electronic devices separate from the biometric device.

**8.** The biometric device of any of claims 1 to 7, wherein the biometric signal is a photoplethysmography (PPG) signal or a glucose measuring signal, and wherein the biometric signal has a smaller bandwidth relative to a frequency of the interference signal.

**9.** The biometric device of any of claims 1 to 8, wherein one or more of the following apply:

a) the Nyquist factor is an odd integer;
b) a sampling rate of the ADC is a function of a frequency of the interference signal
c) a frequency of the interference signal is 50Hz or 60 Hz; and/or
d) the FIR filter is **characterized by** three filter coefficients based on a convolution of another FIR filter **characterized by** two filter coefficients.

**10.** A method of signal attenuation at a biometric device, comprising:

receiving an analog signal from a sensor, the analog signal comprising a biometric signal and an interference signal;
converting the analog signal to a digital signal based on a sampling frequency;
generating a filtered signal from the digital signal via attenuation of the interference signal from the digital signal, wherein the interference signal is attenuated based on a Nyquist factor and an intentional aliasing of the interference signal to at least one Nyquist frequency; and
outputting the filtered signal.

**11.** The method of claim 10, wherein the interference signal is associated with an ambient line frequency of one or more electronic devices separate from the biometric device.

**12.** The method of claim 11, wherein the Nyquist factor is an integer divisor of the ambient line frequency, wherein the intentional aliasing is a result of dividing the ambient line frequency by the integer divisor, and wherein the sampling frequency is a function of the result of dividing the ambient line frequency.

**13.** The method of claims 11 or 12, wherein the analog signal further comprises a flicker signal, wherein a frequency of the flicker signal is twice a frequency of the ambient line frequency, and wherein the method further comprises generating the filtered signal via attenuation of the flicker signal of the digital signal.

**14.** The method of claim 13, wherein one or more of the following apply:

a) the flicker signal is associated with a stroboscopic effect of an artificial light source; and/or;
b) the sampling frequency is set to two-times or four-times the frequency of the interference signal.

**15.** The method of any of claims 10 to 14, wherein the sampling frequency is a function of a frequency of the interference signal.

*FIG. 1*

FIG. 2

FIG. 3

FIG. 4

500

502

receiving an analog signal from a sensor, the analog signal comprising a biometric signal and an interference signal

504

converting the analog signal to a digital signal based on a sampling frequency

506

generating a filtered signal from the digital signal via attenuation of the interference signal from the digital signal, wherein the interference signal is attenuated based on a Nyquist factor and an intentional aliasing of the interference signal to at least one Nyquist frequency

508

generate the filtered signal via attenuation of the flicker signal of the digital signal

510

generate the filtered signal via attenuation of the interference signal based further on: (i) a Nyquist frequency equal to the frequency of the flicker signal, and (ii) a half-Nyquist frequency equal to the frequency of the interference signal

512

outputting the filtered signal

## FIG. 5

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2008 188216 A (KONICA MINOLTA SENSING INC) 21 August 2008 (2008-08-21) * paragraph [0009] - paragraph [0017]; figure 16 * * paragraph [0021] - paragraph [0024] * * paragraph [0031] - paragraph [0032] * * paragraph [0059] - paragraph [0073] * * paragraph [0088] * | 1-15 | INV. A61B5/024 A61B5/145 A61B5/00 |
| A | US 2017/346511 A1 (SHUI TAO [US] ET AL) 30 November 2017 (2017-11-30) * paragraph [0021] - paragraph [0064] * | 1-15 | |
| A | US 2024/032871 A1 (WU MENG-HSUAN [TW]) 1 February 2024 (2024-02-01) * paragraph [0015] - paragraph [0031] * | 1-15 | |

TECHNICAL FIELDS
SEARCHED     (IPC)

A61B

The present search report has been drawn up for all claims

| Place of search | Date of completion of the search | Examiner |
|---|---|---|
| The Hague | 28 April 2025 | Bourquin, Yannyk |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 25 16 3033

28-04-2025

| Patent document cited in search report | Publication date | Patent family member(s) | Publication date |
|---|---|---|---|
| JP 2008188216 A | 21-08-2008 | NONE | |
| US 2017346511 A1 | 30-11-2017 | NONE | |
| US 2024032871 A1 | 01-02-2024 | TW 202406297 A | 01-02-2024 |
| | | US 2024032871 A1 | 01-02-2024 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 60137224